(19) Europäisches Patentamt
European Patent Office
Office européen des brevets

(11) Publication number: **0 168 181**
**B1**

(12) **EUROPEAN PATENT SPECIFICATION**

(45) Date of publication of patent specification: **29.08.90**

(51) Int. Cl.[5]: **C 07 D 487/10,** A 61 K 31/40, C 07 B 57/00 // C07D453/04 ,(C07D487/10, 209:00, 209:00)

(21) Application number: **85304196.0**

(22) Date of filing: **12.06.85**

# (54) Cyclic amides.

(30) Priority: **19.06.84 GB 8415635**

(43) Date of publication of application:
**15.01.86 Bulletin 86/03**

(45) Publication of the grant of the patent:
**29.08.90 Bulletin 90/35**

(84) Designated Contracting States:
**AT BE CH DE FR GB IT LI LU NL SE**

(56) References cited:
**EP-A-0 065 407**
**EP-A-0 115 679**
**JA-S-ik nte**

(73) Proprietor: **IMPERIAL CHEMICAL INDUSTRIES PLC**
**Imperial Chemical House, Millbank**
**London SW1P 3JF (GB)**

(72) Inventor: **Brittain, David Robert**
**23 Springbank Lane**
**Bamford Rochdale Lancs (GB)**

(74) Representative: **Smith, Stephen Collyer et al**
**Imperial Chemical Industries PLC Legal Department: Patents P.O. Box 6 Bessemer Road**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Courier Press, Leamington Spa, England.

EP 0 168 181 B1

**Description**

This invention concerns novel cyclic amides and more particularly a novel 1'-substituted-spiro-[pyrrolidine-3,3'-indoline]-2,2',5-trione which is a potent inhibitor of the enzyme aldose reductase. The invention also concerns pharmaceutical compositions and processes for the manufacture of the novel compound.

The enzyme aldose reductase is responsible in man and other warm-blooded animals for the catalytic conversion of aldoses, for example, glucose and galactose, to the corresponding alditols for example sorbitol and galactitol respectively. Alditols penetrate cell membranes poorly and, once formed, tend to be removed only by further metabolism. As a consequence, alditols tend to accumulate within cells where they are formed, for example in the lens, peripheral nerve tissue and kidney, causing a rise in internal osmotic pressure which may in turn be sufficient to destroy or impair the function of cells themselves. In addition, raised alditol levels may result in abnormal levels of their metabolites which may themselves impair or damage cellular function. However, the enzyme aldose reductase has a relatively low substrate affinity, that is, it is only effective in the presence of relatively large concentrations of aldose. Such large concentrations of aldose are present in the clinical conditions of diabetes (excessive glucose) and galactosemia (excessive galactose). As a consequence, inhibitors of the enzyme aldose reductase are useful in the reduction or prevention of the development of those complications of protracted diabetes or galactosemia which may be due in part to the accumulation of sorbitol or galactitol respectively. Such complications are, for example, macular oedema, cataract, retinopathy, nephropathy or impaired neutral conduction.

It is known from our earlier work that certain 1'-substituted-spiro-[pyrrolidine-3,3'-indoline]-2,2',5-triones of the formula AA (set out hereinafter) wherein Ra is (2—7C)alkyl or (3—7C)alkenyl, naphthylmethyl or cinnamyl optically bearing one or two halogeno nuclear substituents, or Ra is benzyl optionally bearing one or two substituents independently selected from halogeno, (1—4C)alkyl, (1—4C)alkoxy, cyano, nitro and trifluoromethyl, located in the 2, 3, 4 or 5 position; and benzene ring A optionally bears one or two substituents independently selected from halogeno, (1—4C)alkyl, trifluoromethyl and nitro; or a salt thereof with a base affording a pharmaceutically acceptable cation; or a non-toxic, biodegradable precursor thereof; possess useful aldose reductase inhibitory properties (European patent application, publication No. 65407). We have now discovered that a specific compound falling within the above generic definition but not hitherto described, and in particular the laevorotatory optically active form thereof (as measured at 589 nm wavelength in N,N-dimethylformamide), possesses surprisingly potent aldose reductase inhibitory properties, and this is the basis for our invention.

According to the invention there is provided the novel compound 1'-(4-bromo-2-fluorobenzyl)-7'-chloro-spiro[pyrrolidine-3,3'-indoline]-2,2',5-trione of the formula I (set out hereinafter) as its laevorotatory optically active form, or a salt thereof.

The racemic form of the compound of formula I is, as mentioned hereafter, a useful intermediate for said laevorotatory optically active form and, together with its salts, is provided as a feature of the invention.

Particular salts of the novel compound of formula I are, for example, salts which may be used in the isolation, purification or resolution of the free acidic form of said compound, for example the lithium, sodium, potassium, calcium, barium, strontium, aluminium, zinc, iron and silver salts thereof, or salts with optically active forms of organic bases, for example with optically active forms of organic quaternary ammonium hydroxides containing at least one asymmetrically substituted carbon atom, for example with N,N,N-trimethyl-(2-hydroxy-1-methyl-2-phenyl)ethylammonium hydroxide or 1-methylquinidinium hydroxide. Other particular salts of the laevorotatory optically active form of the novel compound of formula I encompassed by the invention are, for example, pharmaceutically acceptable salts of the free acidic form of said compound, for example salts with bases affording physiologically acceptable cations, for example alkali metal and alkaline earth metal salts (such as sodium, potassium, magnesium and calcium salts), aluminium and ammonium salts, and salts with organic amines (such as methylamine, dimethylamine, trimethylamine, piperidine, morpholine, piperazine, ethanolamine, triethanolamine, N-methylglucamine and tetramethylammonium hydroxide).

The novel compound of formula I may be manufactured using any process known in the art for the production of structurally analogous compounds, for example the processes described in our European patent application, publcation No. 65407, generally to give the racemic form which is then resolved to give the required laevorotatory form. Such proceses are provided as a further feature of the invention and are illustrated by the following presently preferred procedures:—

(a) Decarboxylating the acid of the formula II under the influence of heat.

The decarboxylation may be carried out at a temperature in the range, for example, 60—200°C and a suitable solvent or diluent, for example acetic acid, diethanolamine or quinoline (optionally together with copper powder) may conveniently be present.

The starting acid of formula II is conveniently generated *in situ* by hydrolysis of the corresponding derivative of the formula III wherein W is alkoxycarbonyl (such as methoxycarbonyl, ethoxycarbonyl or t-butoxycarbonyl), aralkoxycarbonyl (such as benzyloxycarbonyl), cyano or carbamoyl. The hydrolysis may be carried out using conventional acid or base catalysed conditions, and at a temperature in the range, for example, 40—100°C. When base catalysis is used the acid of formula II must be generated from the salt first

obtained, by careful acidification with an acid such as hydrochloric acid or by addition of carbon dioxide. When acid catalysis is used, the acid of formula II may undergo spontaneous decarboxylation to generate the compound of formula I. Particularly convenient conditions for the *in situ* formulation and subsequent decarboxylation of the acid of formula II are provided by heating a derivative of formula III defined above in a (2—6C)alkanoic acid (such as acetic or propionic acid), in the presence of an inorganic acid (such as hydrogen chloride or hydrogen bromide) and at a temperature in the range, for example, 100—150°C. This process is included as a further feature of the invention.

The starting derivatives of formula III may be obtained by cyclisation of a bifunctional derivative of the formula IV wherein X and Y are preferably cyano and Z is, for example, alkoxycarbonyl such as methoxycarbonyl or ethoxycarbonyl.

The cyclisation is preferably carried out in the presence of an inorganic acid catalyst such as a hydrogen halide, sulphuric acid or polyphosphoric acid, in a suitable solvent or diluent, such as methanol, ethanol, acetic acid or propionic acid and is normally carried out at a temperature in the range, for example 20—120°C. If somewhat higher temperatures are employed, it is possible to carry out the conversion to, and decarboxylation of, the acid of formula II *in situ*, for example by using hydrogen bromide in acetic acid at the boiling point of the reaction mixture. Such a process starting from a derivative of formula IV is provided as a further feature of the invention.

The starting materials of formula IV may be obtained by conventional procedures of organic chemistry well known in the art. Thus, the bifunctional derivatives of the formula IV wherein X is cyano may be obtained by addition of cyanide to an unsaturated compound of the formula V or a geometric isomer thereof, wherein Y and Z have the meanings defined above, for example by reacting an unsaturated compound of formula V with potassium cyanide in methanol at a temperature in the range 10—50°C.

The starting materials of formula V are themselves obtained from 1-(4-bromo-2-fluorobenzyl)-indoline-2,3-dione by condensation with a compound of the formula $Y.CH_2.Z$, wherein Y and Z have the meanings defined above, preferably in the presence of a base catalyst such as piperidine or morpholine, in a suitable solvent such as methanol or ethanol, and at a temperature in the range 60—100°C.

(b) Alkylating a dialkali metal salt of 7′-chlorospiro[pyrrolidine-3,3′-indoline]-2,2′,5-trione by reaction with a 4-bromo-2-fluorobenzyl halide (especially a chloride, bromide or iodide) or a 4-bromo-2-fluorobenzyl alkane- or arene-sulfonate (especially a methane- or *p*-toluene-sulphonate).

The salt is preferably formed *in situ* by using at least two molecular equivalents of base, for example an alkali metal hydride or hydroxide such as sodium or potassium hydride, or sodium or potassium hydroxide. The process is preferably carried out in a suitable polar solvent, for example a (1—4C)alkanol (such as methanol or ethanol), N,N-dimethylformamide or dimethyl sulphoxide, optionally together with a suitable inert diluent, and at a temperature in the range, for example, 10—100°C. It is generally preferred to use only a slight excess of the alkylating agent and to add it dropwise to the reaction mixture in order to minimise the possibility of dialkylation occurring. 7′-Chlorospiro[pyrrolidine-3,3′-indoline]-2,2′-trione may be made analogy with process (a) starting from 7-chloroindoline-2,3-dione.

Process (b) may be conveniently used to produce the required laevorotatory optically active form directly by use of the appropriate optically active form of 7′-chloro-spiro[pyrrolidine-3,3′-indoline)-2,2′,5-trione.

Whereafter the laevorotatory (−) optically active form of the compound of formula I is obtained by reacting the racemic form of said compound with a suitable optically active form of an organic base, for example especially with 1-methylquinidinium hydroxide or methoxide, or with N,N,N-trimethyl-(2-hydroxy-1-methyl-2-phenylethyl)ammonium hydroxide or methoxide, followed by conventional separation of the diastereoisomeric mixture of salts thus obtained, for example, by fractional crystallisation from a suitable solvent, for example acetonitrile, diethyl ether, or 1,2-dimethoxy-ethane, or a mixture thereof, whereafter the laevorotatory optically active form of the said compound may be liberated by treatment of the appropriate salt with acid using a conventional procedure, for example using an aqueous mineral acid, such as dilute hydrochloric acid.

Any unwanted dextrorotatory (+) optically active form of the compound of formula I also obtained, may conveniently be converted to the racemic form (for recycling in the process) by heating, alone or preferably in a suitable solvent or diluent, such as N,N-dimethylformamide, at an elevated temperature, for example 80—280°C.

When a salt of the compound of formula I is required, it may be obtained by a conventional procedure, for example by reaction with the appropriate base or another salt of such a base.

The property of inhibiting the enzyme aldose reductase *in vivo* may be demonstrated in the following standard laboratory test. Thus, rats are made diabetic (as evidenced by severe glucosuria being present) by dosing with streptozotocin. The animals are then dosed daily with the test compound for 2—5 days. The animals are then killed 2—4 hours after the final dose and the eye lenses and/or sciatic nerves are removed. After a standard work-up procedure the residual sorbitol levels in each tissue are determined by gas liquid chromatography after conversion to the poly-trimethylsilyl derivatives. Inhibition of aldose reductase *in vivo* is then assessed by comparing the residual sorbitol levels in tissues from the dosed diabetic group of rats with those of an undosed group of diabetic rats and an undosed, normal group of rats.

The property of inhibiting the enzyme aldose reductase may also be demonstrated *in vitro*. Thus, partially purified aldose reductase is isolated in known manner from bovine lenses. The percentage

inhibition of this enzyme's ability *in vitro* to catalyse the reduction of aldoses to polyhydric alcohols, and particularly to reduce glucose to sorbitol, caused by a test compound is then deermined using standard spectrophotometric methods.

In the above *in vivo* test, an effective dose (ED) may be defined as the oral dose necessary to reduce the residual sorbitol level in the sciatic nerve of a dosed rat to a level similar to that in sciatic nerve in a normal, undosed rat.

In the above *in vivo* test, the laevorotatory form of the compound of formula I has an oral ED of approximately 1.1 mg/kg.

By way of contrast, the laevorotatory form of the structurally similar compound 1'-(4-bromo-2-fluorobenzyl)-7'-fluoro-spiro[pyrrolidine-3,3'-indoline]-2,2',5-trione (which is specifically described in its racemic form in our European patent application, publication No. 65407) has an oral ED in the above *in vivo* test of approximately 5.6 mg/kg.

The laevorotatory form of the compound of formula I will primarily be administered systemically (generally by mouth) to a warm-blooded animal to produce an inhibitory effect on the enzyme aldose reductase, for example at a daily dose of 0.25 to 10 mg/kg. In man it is envisaged that a total daily dose in the range 5 to 250 mg per man will be administered, given if necessary, in divided doses. The compound of formula I may be administered topically for a therapeutic or prophylactic effect mediated by inhibition of the enzyme aldose reductase, for example by topical administration direct to the tissue or organ in which inhibition of the enzyme is required, for example by topical administration to the eye. The precise amount of compound administered will necessarily depend on the formulation used. Thus, for example, when a solution is administered a concentration of the compound containing up to 0.1% by weight will generally be used. Similarly, when an ointment is administered a concentration of the compound of up to 2% by weight will generally be used.

The compounds of formula I will normally be administered to warm-blooded animals in the form of special pharmaceutical formulations. The invention therefore also provides a pharmaceutical composition comprising the laevorotatory optically active form of the compound of formula I, or a salt thereof with a base affording a physiologically acceptable cation, together with a pharmaceutically acceptable diluent or carrier.

The compositions may be in a form suitable for oral administration, for example in the form of a tablet, capsule, granule dispersible powder, syrup, elixir, emulsion, suspension or gel; for parenteral administration, for example in the form of a sterile injectable aqueous suspension or solution, or oily solution or suspension; for rectal administration, for example in the form of a suppository; or for topical administration, for example especially to the eye, in the form of an ointment, gel or sterile solution buffered at an ophthalmically acceptable pH, for example in the range pH 7.0—7.6.

Topical formulations may be administered to the eye of an animal, for example man or dogs, requiring treatment for diabetic cataracts or retinopathy in a conventional manner, for example using a drop or eyewash topical formulation.

The compositions may also contain one or more other agents which are known to have a useful effect in the treatment of diabetes or galactosemia, for example a hypoglycaemic agent as tolbutamide, chlorpropamide, or glybenclamide.

The invention will now be illustrated by the following non-limiting Examples in which, unless otherwise stated:—

(i) all evaporations were carried out by rotary evaporation *in vacuo*:

(ii) all operations were carried out at room temperature, that is in the range 18—26°C;

(iii) the purity of chemical products was assessed by nuclear magnetic resonance spectroscopy, thin layer chromatographic analysis and/or microanalysis;

(iv) petroleum ether (b.p. 60—80°C) is referred to as "petrol 60—80"; and

(v) yields are for illustration only and are not necessarily the maximum attainable by diligent process development.

Example 1

The racemic form of 1'-(4-bromo-2-fluorobenzyl)-7'-chloro-spiro[pyrrolidine-3,3'-indoline]-2,2',5-trione (A) (3.0 g, 6.86 mM) was added to a methanolic solution (916 ml) containing an equivalent quantity of 1-methylquinidinium methoxide to give a clear solution. The solvent was removed by evaporation below 50°C and the residue was recrystallised three times from a 1:1 v/v mixture of ether and acetonitrile to give the 1-methylquinidinium salt of the laevorotatory (−) form of A as a crystalline solid (1.1 g), m.p. 227°C, $^{20}[\alpha]_{589}$ + 93.7° (c = 1.0, 50% aqueous pyridine). This salt was then dissolved in methanol and the solution acidified with 2M hydrochloric acid. The precipitate which formed was separated by filtration, washed with water and recrystallised from 1:1 v/v ethyl acetate/petrol 60—80 to give the laevorotatory form of 1'-(4-bromo-2-fluorobenzyl)-7'-chloro-spiro[pyrrolidine-3,3'-indoline]-2,2',5-trione (0.3 g), m.p. 210—211°C, $^{20}[\alpha]_{589}$ − 35.1° (c = 1; N,N-dimethylformamide).

The starting racemic form of A was obtained as follows:—

(i) A suspension of 7-chloro-indoline-2,3-dione (15 g) and potassium carbonate (15 g) in N,N-dimethylformamide (100 ml) was stirred and treated with a solution of 4-bromo-2-fluorobenzyl bromide (24.7 g) in chlorobenzene (100 ml). The mixture was stirred at 90°C for five hours, cooled to ambient temperature, and

diluted with a mixture of water (500 ml) and petrol 60—80 (500 ml). The aqueous phase was adjusted to pH 3 with 10M hydrochloric acid. The mixture obtained was separated by filtration. The solid residue was washed with petrol 60—80, then with water, and recrystallised from ethanol to give 1-(4-bromo-2-fluorobenzyl)-7-chloro-indoline-2,3-dione (B) (15.5 g), m.p. 164—166°C.

(ii) A mixture of B (73.7 g), methyl cyanoacetate (21.8 g) and piperidine (0.5 ml) in methanol (500 ml) was heated under reflux for 4 hours. The mixture was then cooled to 0—5°C. The solid which had formed was collected by filtration and washed with hexane to give methyl 2-[1-(4-bromo-2-fluorobenzyl)-7-chloro-2-oxo-3-indolinyl]-2-cyanoacetate (C) (74.8 g), m.p. 138—140°C.

(iii) Potassium cyanide (16.5 g) was added to a solution of C (54.0 g) in a mixture of methanol (750 ml) and water (250 ml). The mixture was stirred for 4 hours, then diluted with methanol (250 ml) and treated with decolourising charcoal (3.0 g). The resultant mixture was stirred for 30 minutes. The charcoal was then removed by filtration through diatomaceous earth. The filtrate was diluted with water (1.51) and then acidified to pH 2 with concentrated hydrochloric acid. The solid which had formed was collected by filtration, washed with water and air-dried to give methyl 2-[1-(4-bromo-2-fluorobenzyl)-7-chloro-3-cyano-2-oxo-3-indolinyl]-2-cyanoacetate (D) (45.9 g), m.p. 146—148°C.

(iv) A mixture of D (30 g) with a 48% w/w solution (300 ml) of hydrogen bromide in acetic acid (300 ml) was heated under reflux for 4 hours. The solution obtained was then cooled and poured into water. The precipitate which formed was collected by filtration, washed with water and air-dried to give the impure racemic form of 1'-(4-bromo-2-fluorobenzyl)-7'-chloro-spiro[pyrrolidine-3,3'-indoline]-2,2',5-trione (A) (27.1 g). This material was purified by column chromatography on silica gel (450 g) using 10% v/v ethyl acetate/petrol 60—80 to elute impurities. Elution with 25% v/v ethyl acetate/petrol 60—80 gave the pure racemic form of A (9.8 g), m.p. 216—218°C.

The preparation of the starting 1-methylquinidinium methoxide is illustrated as follows:—

A suspension of anhydrous quinidine (0.8 g) and anhydrous potassium carbonate (2.0 g) in acetone (37 ml) was stirred for 30 minutes. The mixture was then evaporated below 35°C. The residue was added to a mixture of water (100 ml) and chloroform (100 ml), and stirred vigorously for 10 minutes. The solid residue was separated by filtration and recrystallised from ethanol to give 1-methylquinidinium iodide (e) as a white solid (0.6 g), m.p. 245—247°C. This iodide was then dissolved in methanol and the solution obtained was passed through a column of anion exchanging resin ["Amberlite" (Trade-mark) IRA 401, freshly converted into the hydroxide form] using methanol as the eluant. The basic strength of the eluate was then obtained by a standard titration with 0.2M hydrochloric acid.

## Example 2

(All parts by weight)

A mixture of the laevorotatory form of 1'-(4-bromo-2-fluorobenzyl)-7'-chloro-spiro[pyrrolidine-3,3'-indoline]-2,2',5-trione (50 parts), lactose (27 parts) and maize starch (20 parts), was stirred throroughly and a paste formed from maize starch (2 parts) and water (40 parts) was added and thoroughly mixed in. The resultant mass was passed through a 16 mesh screen, then dried at 60°C and passed through a 20 mesh screen. Magnesium stearate (1 part) was added to the granules obtained, and the whole compressed by conventional means into tablets, containing 10, 20, 50 or 100 mg of active ingredient, suitable for oral administration for therapeutic purposes.

Chemical Formulae:

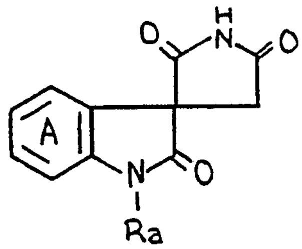

AA

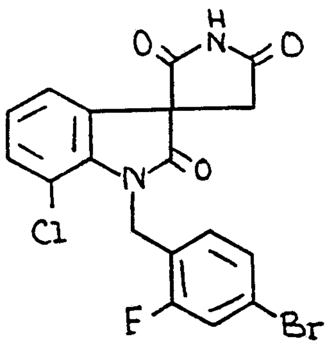

I

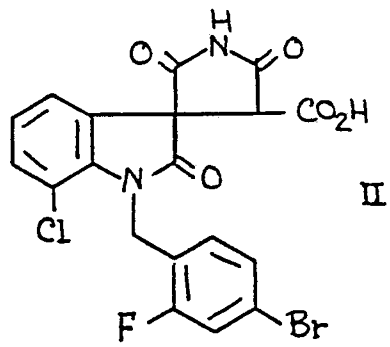

II

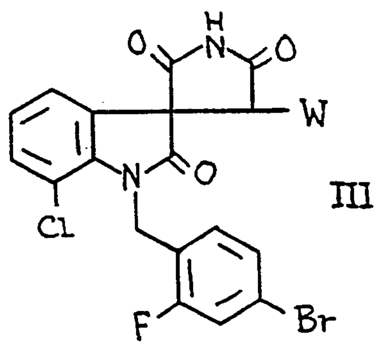

III

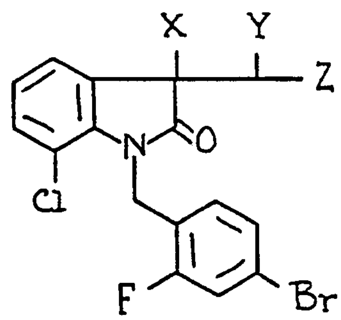

IV

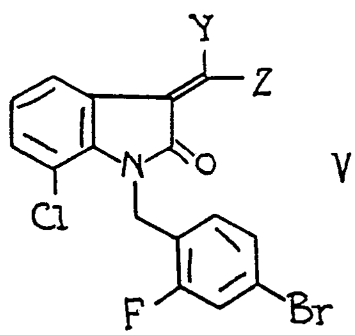

V

**Claims for the Contracting States: BE CH DE FR GB IT LI LU NL SE**

1. 1'-(4-Bromo-2-fluorobenzyl)-7'-chloro-spiro[pyrrolidine-3,3'-indoline]-2,2',5-trione in laevorotatory optically active form, or a salt thereof.

2. A salt as claimed in claim 1 which is selected from lithium, sodium, potassium, calcium, barium, strontium, aluminium, zinc, iron and silver salts, and from salts with optically active forms of organic quaternary ammonium hydroxides.

3. A salt as claimed in claim 1 selected from salts with bases affording physiologically acceptable cations.

4. A salt as claimed in claim 3 selected from lithium, sodium, potassium, magnesium, calcium, aluminium and ammonium salts, and from salts with organic amines affording physiologically acceptable cations.

5. 1'-(4-Bromo-2-fluorobenzyl)-7'-chloro-spiro[pyrrolidine-3,3'-indoline]-2,2',5-trione in racemic form.

6. A pharmaceutical composition comprising 1'-(4-bromo-2-fluorobenzyl)-7'-chloro-spiro[pyrrolidine-3,3'-indoline]-2,2',5-trione in laevorotatory optically active form, or a salt thereof with a base affording a physiologically acceptable cation.

7. A composition as claimed in claim 6 for use in the inhibition of the enzyme aldose reductase.

8. A composition as claimed in claim 6 or 7 which is in a form suitable for oral, parenteral, rectal or topical administration.

9. A process for the manufacture of the compound 1'-(4-bromo-2-fluorobenzyl)-7'-chloro-spiro-[pyrrolidine-3,3'-indoline]-2,2',5-trione in laevorotatory optically active form, or a salt thereof, which comprises the steps of:—

(i) forming the racemic form of said compound by:—

(a) decarboxylating the acid of the formula II

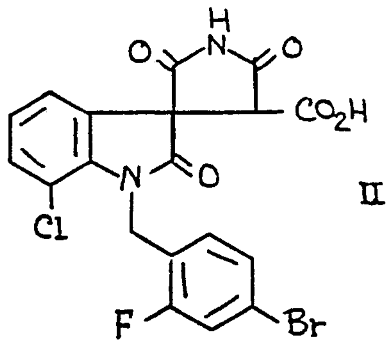

II

under the influence of heat; or

(b) alkylating a dialkali metal salt of 7'-chloro-spiro[pyrrolidine-3,3'-indoline]-2,2',5-trione by reaction with a 4-bromo-2-fluorobenzyl halide, alkane-sulphonate or arene-sulphonate; and

(ii) resolving the racemic form of said compound by reacting the said racemic form with a suitable optically active organic base, followed by conventional separation of the diastereoisomeric mixture of salts thus obtained and liberation of the required laevorotatory optically active form of said compound by acidification of the diastereoisomeric salt;

whereafter, when a salt is required, the laevorotatory form of said compound is reacted with the appropriate base.

10. A process as claimed in claim 9 wherein in step (ii) the optically active base is 1-methylquinidinium hydroxide or methoxide.

**Claims for the Contracting State: AT**

1. A process for the manufacture of 1'-(4-bromo-2-fluorobenzyl)-7'-chloro-spiro[pyrrolidine-3,3'-indoline]-2,2',5-trione in laevorotatory optically active form, or a salt thereof; characterised by carrying out the steps of:—

(i) forming the racemic form of said compound by:—

(a) decarboxylating the acid of the formula II

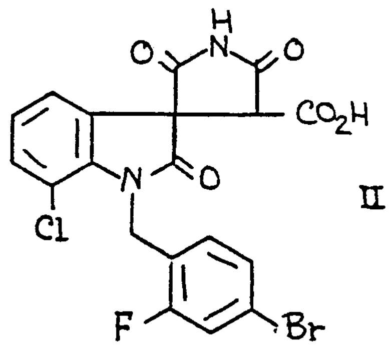

II

under the influence of heat; or

(b) alkylating a dialkali metal salt of 7'-chloro-spiro[pyrrolidine-3,3'-indoline]-2,2',5-trione by reaction with a 4-bromo-2-fluorobenzyl halide, alkane-sulphonate or arene-sulphonate; and

(ii) resolving the racemic form of said compound by reacting the said racemic form with a suitable optically active organic base, followed by conventional separation of the diastereoisomeric mixture of salts thus obtained and liberation of the required laevorotatory optically active form of said compound by acidification of the diastereoisomeric salt;

whereafter, when a salt is required, the laevorotatory form of said compound is reacted with the appropriate base.

2. A process according to claim 1 characterised in that in (a) the acid of formula II is produced *in situ* by hydrolysis of a compound of formula III

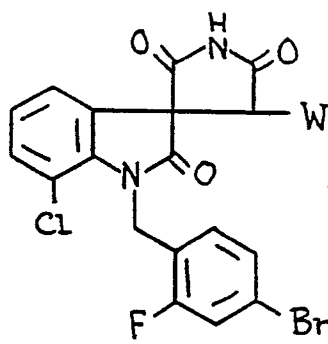

III

wherein W is alkoxycarbonyl, aralkoxycarbonyl, cyano or carbamoyl.

3. A process according to claim 1 characterised in that in (a) the acid of formula II is produced *in situ* by acid catalysed cyclisation and hydrolysis of a bifunctional derivative of formula IV

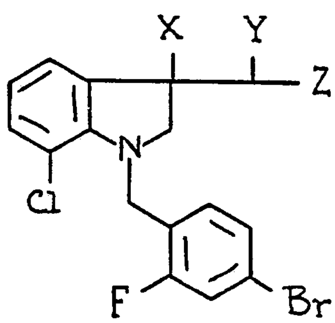

IV

wherein X and Y are cyano and Z is alkoxycarbonyl at a temperature in the range 100—500°C.

4. A process according to claim 3 characterised in that a mixture of hydrogen bromide in acetic acid is used to provide the acid catalysis.

5. A process according to any of claims 1—4 characterised in that in step (ii) the optically active base is 1-methylquinidinium hydroxide or methoxide.

**Patentansprüche für die Vertragsstaaten: BE CH DE FR GB IT LI LU NL SE**

1. 1'-(4-Bromo-2-fluorobenzyl)-7'-chloro-spiro[pyrrolidin-3,3'-indolin]-2,2',5-trion in der linksdrehenden optisch aktiven Form oder ein Salz davon.

2. Salz nach Anspruch 1, welches ausgewählt ist aus den Lithium-, Natrium-, Kalium-, Calcium-, Barium-, Strontium-, Aluminium-, Zink-, Eisen- und Silber-Salzen und aus den Salzen mit optisch aktiven Formen von organischen quaternären Ammoniumhydroxiden.

3. Salz nach Anspruch 1, welches ausgewählt ist aus Salzen mit Basen, die physilogisch zulässige Kationen liefern.

4. Salz nach Anspruch 3, welches ausgewählt ist aus den Lithium-, Natrium-, Kalium-, Magnesium-, Calcium-, Aluminium- und Ammonium-Salzen und aus den Salzen mit organischen Aminen, die physiologisch zulässige Kationen liefern.

5. 1'-(4-Bromo-2-fluorobenzyl)-7'-chloro-spiro[pyrrolidin-3,3'-indolin]-2,2',5-trion in racemischer Form.

6. Pharmazeutische Zusammensetzung, welche 1'-(4-Bromo-2-fluorobenzyl)-7'-chloro-spiro-[pyrrolidin-3,3'-indolin]-2,2',5-trion in der linksdrehenden optisch aktiven Form oder ein Salz davon mit einer Base, die ein physiologisch zulässiges Kation liefert, enthält.

7. Zusammensetzung nach Anspruch 6 für die Verwendung bei der Inhibierung des Enzyms Aldosereductase.

8. Zusammensetzung nach Anspruch 6 oder 7, welche eine Form aufweist, die sich für orale, parenterale, rectale oder topische Verabreichung eignet.

9. Verfahren zur Herstellung der Verbindung 1'-(4-Bromo-2-fluorobenzyl)-7'-chloro-spiro[pyrrolidin-3,3'-indolin]-2,2',5-trion in der linksdrehenden optisch aktiven Form oder eines Salzes davon, welches die folgenden Stufen umfaßt:

(i) Herstellen der racemischen Form dieser Verbindung durch

(a) Decarboxylierung der Säure der Formel II

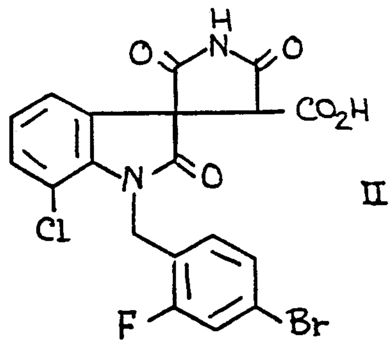

II

unter dem Einfluß von Wärme oder

(b) Alkylierung eines Dialkalimetall-Salzes von 7'-Chloro-spiro[pyrrolidin-3,3'-indolin]-2,2',5-trion durch Reaktion mit einem 4-Bromo-2-fluorobenzylhalogenid, Alkan-sulfonat oder Aren-sulfonat; und

(ii) Trennen der racemischen Form dieser Verbindung durch Umsetzen dieser racemischen Form mit einer geeigneten optisch aktiven organischen Base, anschließende übliche Trennung des so erhaltenen diastereoisomeren Salzgemisches und Freisetzen der gewünschten linksdrehenden optisch aktiven Form dieser Verbindung durch Ansäuerung des diastereoisomeren Salzes;

worauf, wenn ein Salz gewünscht wird, die linksdrehende Form dieser Verbindung mit der entsprechenden Base umgesetzt wird.

10. Verfahren nach Anspruch 9, bei welchem in der Stufe (ii) als optische aktive Base 1-Methylchinidinium-hydroxid oder -methoxid verwendet wird.

**Patentansprüche für den Vertragsstaat: AT**

1. Verfahren zur Herstellung von 1'-(4-Bromo-2-fluorobenzyl)-7'-chloro-spiro[pyrrolidin-3,3'-indolin]-2,2',5-trion in der linksdrehenden optisch Form aktiven Form oder eines Salzes davon, dadurch gekennzeichnet, daß man die folgenden Stufen ausführt:
(i) Herstellen der racemischen Form dieser Verbindung durch
(a) Decarboxylierung der Säure der Formel II

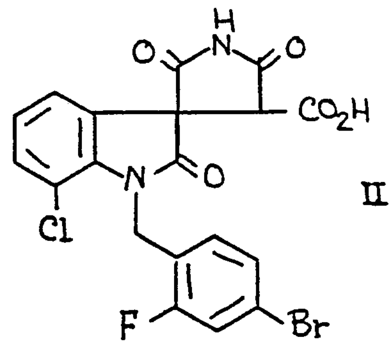

II

unter dem Einfluß von Wärme oder
(b) Alkylierung eines Dialkalimetall-Salzes von 7'-chloro-spiro[pyrrolidin-3,3'-indolin]-2,2',5-trion durch Reaktion mit einem 4-Bromo-2-fluorobenzylhalogenid, Alkan-sulfonat oder Aren-sulfonat; und
(ii) Trennen der racemischen Form dieser Verbindung durch Umsetzen dieser racemischen Form mit einer geeigneten optisch aktiven organischen Base, anschließende übliche Trennung des so erhaltenen diastereoisomeren Salzgemisches und Freisetzen der gewünschten linksdrehenden optisch aktiven Form dieser Verbindung durch Ansäuerung des diastereoisomeren Salzes;
worauf, wenn ein Salz gewünscht wird, die linksdrehende Form dieser Verbindung mit der entsprechenden Base umgesetzt wird.

2. Verfahren nach Anspruch 1, dadurch gekennzeichnet, daß bei (a) die Saure der Formel II in situ hergestellt wird durch Hydrolyse der Verbindung der Formel III

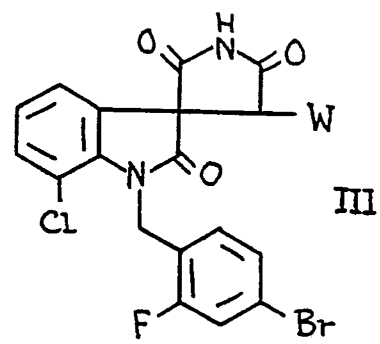

III

worin W für Alkoxycarbonyl, Aralkoxycarbonyl, Cyano oder Carbamoyl steht.

3. Verfahren nach Anspruch 1, dadurch gekennzeichnet, daß in (a) die Säure der Formel II in situ hergestellt wird durch katalysierte Cyclisierung und Hydrolyse eines bifunktionellen Derivates der Formel IV

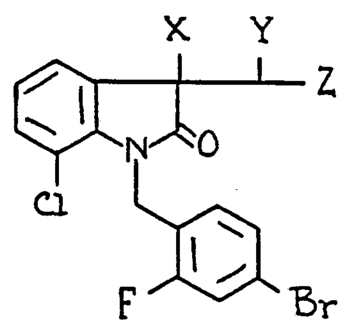

IV

worin X und Y für Cyano stehen und Z für Alkoxycarbonyl steht, bei einer Temperatur im Bereich von 100—150°C.

4. Verfahren nach Anspruch 3, dadurch gekennzeichnet, daß ein Gemisch aus Bromwasserstoff in Essigsäure für die Durchführung der sauren Katalyse verwendet wird.

5. Verfahren nach einem der Ansprüche 1 bis 4, dadurch gekennzeichnet, daß in Stufe (ii) die optisch aktive Base 1-Methylchinidinium-hydroxid oder -methoxid ist.

**Revendications pour les Etats contractants: BE CH DE FR GB IT LI LU NL SE**

1. 1'-(4-bromo-2-fluorobenzyl)-7'-chloro-spiro[pyrrolidino-3,3'-indolino]-2,2',5-trione, sous la forme lévogyre optiquement active, ou un de ses sels.

2. Sel suivant la revendication 1, qui est choisi entre les sels de lithium, de sodium, de potassium, de calcium, de baryum, de strontium, d'aluminium, de zinc, de fer et d'argent, et entre des sels obtenus avec des formes optiquement actives d'hydroxydes d'ammonium quaternaire organiques.

3. Sel suivant la revendication 1, choisi entre des sels formés avec des bases donnant des cations physiologiquement acceptables.

4. Sel suivant la revendication 3, choisi entre des sels de lithium, de sodium, de potassium, de magnésium, de calcium, d'aluminium et d'ammonium, et entre des sels formés avec des amines organiques donnant des cations physiologiquement acceptables.

5. La 1'-(4-bromo-2-fluorobenzyl)-7'-chloro-spiro[pyrrolidino-3,3'-indolino]-2,2',5-trione, sous forme racémique.

6. Composition pharmaceutique comprenant de la 1'-(4-bromo-2-fluorobenzyl)-7'-chloro-spiro-[pyrrolidino-3,3'-indolino]-2,2',5-trione sous la forme optiquement active lévogyre, ou un de ses sels formé avec une base donnant un cation physiologiquement acceptable.

7. Composition suivant la revendication 6, destinée à être utilisée dans l'inhibition l'enzyme appelé aldose-réductase.

8. Composition suivant la revendication 6 ou 7, qui est sous une forme convenable pour l'administration orale, parentérale, rectale ou topique.

9. Procédé de production de 1'-(4-bromo-2-fluorobenzyl)-7'-chloro-spiro[pyrrolidino-3,3'-indolino]-2,2',5-trione sous la forme optiquement active lévogyre, ou d'un de ses sels, qui comprend les étapes consistant:

(i) à préparer la forme racémique dudit composé par:

(a) décarboxylation de l'acide de formule II

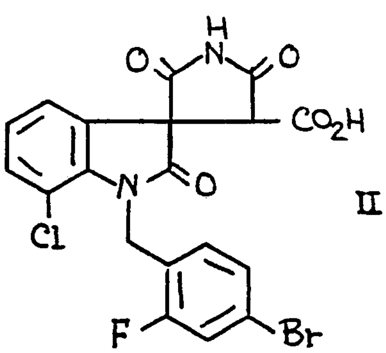

II

sous l'influence de la chaleur; ou

(b) alkylation d'un sel, à deux atomes de métal alcalin, de 7'-chloro-spiro[pyrrolidino-3,3'-indolino]-2,2',5-trione par réaction avec un halogénure, un alcanesulfonate ou un arènesulfonate de 4-bromo-2-fluorobenzyle; et

(ii) à soumettre à une résolution la forme racémique dudit composé par réaction de ladite forme racémique avec une base organique optiquement active convenable, puis par une séparation classique du mélange de sels diastéréoisomères ainsi obtenu et par libération de la forme optiquement active lévogyre requise dudit composé par acidification du sel diastéréo-isomère;

la forme lévogyre dudit composé, lorsqu'un sel est requis, étant ensuite amenée à réagir avec la base appropriée.

10. Procédé suivant la revendication 9, dans lequel dans l'étape (ii), la base optiquement active est l'hydroxyde ou le méthylate de 1-méthylquinidinium.

**Revendications pour l'Etat contractant: AT**

1. Procédé de production de 1'-(4-bromo-2-fluorobenzyl)-7'-chloro-spiro[pyrrolidino-3,3'-indolino]-2,2',5-trione sous la forme optiquement active lévogyre, ou d'un de ses sels; caractérisé en ce qu'il consiste à mettre en oeuvre les étapes consistant:

(i) à préparer la forme racémique dudit composé par:

(a) décarboxylation de l'acide de formule II

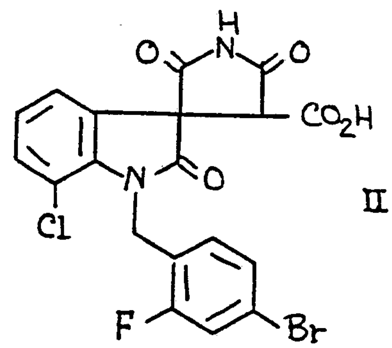

II

sous l'influence de la chaleur; ou

(b) alkylation d'un sel, à deux atomes de métal alcalin, de 7'-chloro-spiro[pyrrolidino-3,3'-indolino]-2,2',5-trione par réaction avec un halogénure, un alcanesulfonate ou un arènesulfonate de 4-bromo-2-fluorobenzyle; et

(ii) à soumettre à une résolution la forme racémique dudit composé par réaction de ladite forme racémique avec une base organique optiquement active convenable, puis par une séparation classique du mélange de sels diastéréoisomères ainsi obtenu et par libération de la forme optiquement active lévogyre requise dudit composé par acidification du sel diastéréo-isomère;

la forme lévogyre dudit composé, lorsqu'un sel est requis, étant ensuite amenée à réagir avec la base appropriée.

2. Procédé suivant la revendication 1, caractérisé en ce que, dans l'étape (a), l'acide de formule II est produit in situ par hydrolyse d'un composé de formule III

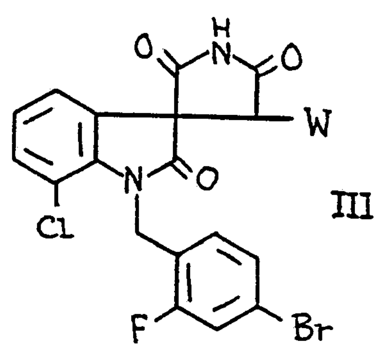

III

dans laquelle W représente un groupe alkoxycarbonyle, aralkoxycarbonyle, cyano ou carbamoyle.

3. Procédé suivant la revendication 1, caractérisé en ce que, dans l'étape (a), l'acide de formule II est produit in situ par cyclisation, catalysée par un acide, et hydrolyse d'un dérivé bifonctionnel de formule IV

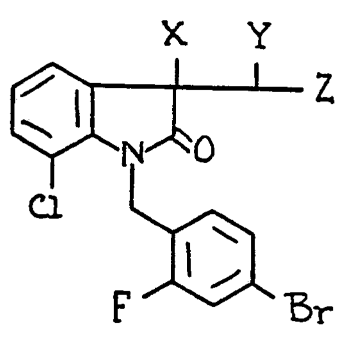

IV

dans laquelle X et Y représentent des groupes cyano et Z représente un groupe alkoxycarbonyle, à une température comprise dans l'intervalle de 100 à 150°C.

Procédé suivant la revendication 3, caractérisé en ce qu'un mélange d'acide bromhydrique et d'acide acétique est utilisé pour effectuer la catalyse acide.

5. Procédé suivant l'une quelconque des revendications 1 à 4, caractérisé en ce que, dans l'étape (ii), la base optiquement active est l'hydroxyde ou le méthylate de 1-méthylquinidinium.